# EUROPEAN PATENT SPECIFICATION

(11) **EP 1 541 151 B1**
(45) Date of publication and mention of the grant of the patent: **22.02.2012**
(21) Application number: 03791404.1
(22) Date of filing: 29.08.2003
(51) Int. Cl.: A61K 31/5575, A61K 45/06, A61K 31/437, A61K 31/4409, A61K 31/4725, A61K 31/5377, A61K 31/553, A61P 27/06, A61P 43/00

(54) **REMEDY FOR GLAUCOMA COMPRISING Rho KINASE INHIBITOR AND PROSTAGLANDINS**
MITTEL ZUR BEHANDLUNG VON GLAUKOM MIT EINEM Rho-KINASE-HEMMER UND PROSTAGLANDINEN
TRAITEMENT CONTRE LE GLAUCOME A BASE D'INHIBITEURS DE RHO KINASE ET DE PROSTAGLANDINES

(30) Priority: 29.08.2002 JP 2002250223
(43) Date of publication of application: 15.06.2005
(60) Divisional application: 10192265.6
(73) Proprietor: SANTEN PHARMACEUTICAL CO., LTD., Higashiyodogawa-ku, Osaka-shi, Osaka 533-8651 (JP)
(72) Inventor: NAKAJIMA, Tadashi, SANTEN PHARMACEUTICAL CO., LTD., Ikoma-shi, Nara 630-0101 (JP); MATSUGI, Takeshi, SANTEN PHARMACEUTICAL CO., LTD., Ikoma-shi, Nara 630-0101 (JP); HARA, Hideaki, SANTEN PHARMACEUTICAL CO., LTD., Ikoma-shi, Nara 630-0101 (JP)
(74) Representative: Peaucelle, Chantal
(86) International application number: PCT/JP2003/011004
(87) International publication number: WO 2004/019951

(56) References cited:
- EP-A1- 0 286 903
- EP-A2- 0 308 135
- EP-A2- 0 639 563
- WO-A-00/25771
- WO-A1-00/09162
- WO-A1-90/02553
- WO-A1-94/06433
- WO-A1-97/23222
- WO-A1-98/06433
- ZHOU YAN ET AL: "Nonsteroidal anti-inflammatory drugs can lower amyloidogenic Abeta42 by inhibiting Rho." SCIENCE (WASHINGTON D C), vol. 302, no. 5648, 14 November 2003 (2003-11-14), pages 1215-1217, XP002436030 ISSN: 0036-8075
- HONJO MEGUMI ET AL: "Effects of Rho-associated protein kinase inhibitor Y-27632 on intraocular pressure and outflow facility" INVESTIGATIVE OPHTHALMOLOGY & VISUAL SCIENCE, ASSOCIATION FOR RESEARCH IN VISION AND, US, vol. 42, no. 1, January 2001 (2001-01), pages 137-144, XP002343585 ISSN: 0146-0404
- HONJO M ET AL: "Effects of Protein Kinase Inhibitor, HA1077, on Intraocular Pressure and Outflow Facility in Rabbit Eyes" ARCHIVES OF OPHTHALMOLOGY, vol. 119, no. 8, August 2001 (2001-08), pages 1171-1178, XP002999418 ISSN: 0003-9950
- DATABASE WPI Week 200060 Derwent Publications Ltd., London, GB; AN 2000-628321 XP002436032 & WO 00/57914 A1 (SANTEN PHARM CO LTD) 5 October 2000 (2000-10-05)
- CLARK A F ET AL: "Advances in glaucoma therapeutics" EXPERT OPINION ON EMERGING DRUGS 2002 UNITED KINGDOM, vol. 7, no. 1, May 2002 (2002-05), pages 141-163, XP009079945 ISSN: 1472-8214

## Description

### Technical Field

The present invention relates to a therapeutic agent for glaucoma comprising the combination of a Rho kinase inhibitor and a prostaglandin.

### Background Art

Glaucoma is an intractable ocular disease with a risk of blindness, involving the increase of intraocular pressure due to various factors and by disordering internal tissues of eyeballs (retina, an optic nerve and the like). A general method of treating glaucoma is intraocular pressure reduction therapy, which is exemplified by pharmacotherapy, laser therapy, surgery therapy and the like.

For the pharmacotherapy, drugs such as sympathomimetic agents (nonselective stimulants such as epinephrine, α ₂ stimulants such as apraclonidine), sympatholytic agents (β-blockers such as timolol and befunolol, α ₁-blokers such as bunazosin hydrochloride), parasympathomimetic agents (pilocarpine and the like), carbonic anhydrase inhibitors (acetazolamide and the like) and prostaglandins (isopropyl unoprostone, latanoprost, travoprost, bimatoprost and the like) have been used.

Recently, a Rho kinase inhibitor was found to serve as a therapeutic agent for glaucoma based on a new mechanism of action (WO 00/09162). Invest. Ophthalmol. & Vis. Sci., 42 (1), 137-144 (2001) discloses that the Rho kinase inhibitor increases the aqueous humor outflow from a trabecular meshwork outflow pathway thereby reducing intraocular pressure, and Invest. Ophthalmol. & Vis. Sci., 42 (1), 137-144 (2001) and Invest. Ophthalmol. & Vis. Sci., 42 (5), 1029-1037 (2001) suggest that the mechanism of action is reconstruction of cytoskeleton in trabecular meshwork cells.

Combined use of drugs having actions of reducing intraocular pressure to treat glaucoma has already been studied and there are some reports on the studies. For example, Japanese Patent No. 2726672 reports combined administration of the sympatholytic agent with prostaglandins. WO 02/38158 discloses a method of treating glaucoma by administering some drugs having actions of reducing intraocular pressure in combination to eyes.

However, any reports do not describe the Rho kinase inhibitor at all, and naturally, there is no description concerning advantageous effects brought about by combining the inhibitor with prostaglandins, either.

As mentioned above, neither study nor report has been made concerning therapeutic effects on glaucoma obtained by combining the Rho kinase inhibitor with prostaglandins, so far.

### Disclosure of the Invention

It is a very interesting subject to find utility as a therapeutic agent for glaucoma due to a combination of a Rho kinase inhibitor and a prostaglandin.

Studying precisely effects due to the combination of a Rho kinase inhibitor and a prostaglandin, the present inventors found that an action of reducing intraocular pressure is increased and/or persistence of the action is improved by combining these drugs compared with a case where each drug is used alone and consequently completed the present invention. Detailed test methods and their effects are described later in the section of "Pharmacological Tests". A remarkable increase in action of reducing intraocular pressure and/or remarkable improvement of persistence of the action was observed by combining a Rho kinase inhibitor with a prostaglandin.

The present invention relates to a therapeutic agent for the use in the treatment of glaucoma comprising the combination of a Rho kinase inhibitor,(R)·(+)-N-(1H-pyrrolo[2,3.b]pyridin-4-yl)-4-(1-aminoethy)-benzamide and latanoprost. These drugs each other complement and/or enhance their actions.

For the administration mode, each of the Rho kinase inhibitor and the prostaglandin can be in a separate preparation and these drugs can be administered in combination. Alternatively, these drugs can be formulated in a single preparation to be administered. In other words, these drugs can be administered in mixture.

The Rho kinase inhibitors and prostaglandins of the present invention include salts thereof. When these compounds have a basic group such as an amino group, they can be salts with an inorganic acid such as hydrochloric acid or nitric acid or with an organic acid with oxalic acid, succinic acid or acetic acid. When they have an acidic group such as a carboxyl group, they can be salts with an alkali metal such as sodium or potassium or with an alkaline earth metal such as calcium.

The Rho kinase inhibitor used in the present invention is a compound which inhibits serine/threonine kinase activated with activation of Rho. (R)-(+)-N-(1H-pyrrolo[2,3-b]pyridin-4-yl)-4-(1-aminoethyl)-benzamide is disclosed in WO 00/09162. On the other hand, the prostaglandin has the action of reducing intraocular pressure and utility in treating glaucoma can be used. Latanoprost is described in Published Japanese Translation of PCT No. 501025/1991.

Examples of glaucoma in the present invention are primary open angle glaucoma, normal intraocular tension glaucoma, hypersecretion glaucoma, ocular hypertension, acute angle-closure glaucoma, chronic closed angle glaucoma, combined-mechanism glaucoma, corticosteroid glaucoma, amyloid glaucoma, neovascular glaucoma, malignant glaucoma, capsular glaucoma, plateau iris syndrome and the like.

The invention also relates to the use of a combination of (R)-(+)-N-(1H-pyrrolo[2,3-b]pyridin-4-yl)-4-(1-aminoethyl)-benzamide and latanoprost in the manufacture of a therapeutic agent for glaucoma.

It also relates to the use of said combination in the manufacture of a therapeutic agent for glaucoma, characterized in that their actions are complemented and/or enhance each other.

To carry out the present invention, preparations can be two preparations prepared by formulating the Rho kinase inhibitor and the prostaglandin separately or one preparation prepared by mixing these ingredients. Particular techniques are unnecessary for the formulation, and the preparations can be prepared using widely-used techniques. A preferred method of administration is eye topical administration, and a preferred dosage form is an ophthalmic solution or an eye ointment.

When the Rho kinase inhibitor and the prostaglandin are formulated in preparations separately, each preparation can be prepared according to known methods. For example, the Rho kinase inhibitor can be formulated in preparations by referring to Formulation Examples described in the above-mentioned International Publication WO 00/09162. Prostaglandin can be formulated in preparations by referring to Formulation Examples described in the above-mentioned Published Japanese Translation of PCT No. 501025/1991 and as commercially available preparations thereof.

The formulation containing the Rho kinase inhibitor and the prostaglandin in mixture can be also prepared according to known methods. The ophthalmic solutions can be prepared, using isotonic agents such as sodium chloride and concentrated glycerin; buffers such as sodium phosphate buffer and sodium acetate buffer; surfactants such as polyoxyethylene sorbitan monooleate, stearate polyoxyl 40, and polyoxyethylene hardened castor oil; stabilizers such as sodium citrate and sodium edetate; and preservatives such as benzalkonium chloride and paraben, as needed. The pH should be within an ophthalmologically acceptable range and is preferably within a range of pH 4 to pH 8. For reference, a formulation example thereof is described below in the section of Example. However, the formulation example never limits the scope of the invention.

The doses of Rho kinase inhibitor and prostaglandin can be determined depending on the symptom and age of patients, the dosage form, the administration route and the like. The case of instillation is briefly described below. The dose of the Rho kinase inhibitor varies depending on the drug type. The Rho kinase inhibitor can be administered generally within 0.025 to 10,000 µg daily from once to several times a day. The dose can be appropriately raised or lowered depending on the age and symptom of patients and the like.

The usual daily dose of the prostaglandin is within a range of 0.1 to 1,000 µg, which can be administered from once to several times a day. More specifically, latanoprost is generally administered at a daily dose of 1 to 5 µg and a daily dose of 30 to 300 µg, respectively. Depending on the age and symptom of patients and the like, the doses are varied.

These doses are also applicable to the administration of the combination of the Rho kinase inhibitor and the prostaglandin. In case that the Rho kinase inhibitor and the prostaglandin are to be administrated in one formulation, the formulation should be prepared by selecting the mixing ratio of two drugs appropriately so that their daily doses might not excess each dose of the separate drugs. The mixed formulation can be administered from once to several times daily.

### Brief Description of Drawing

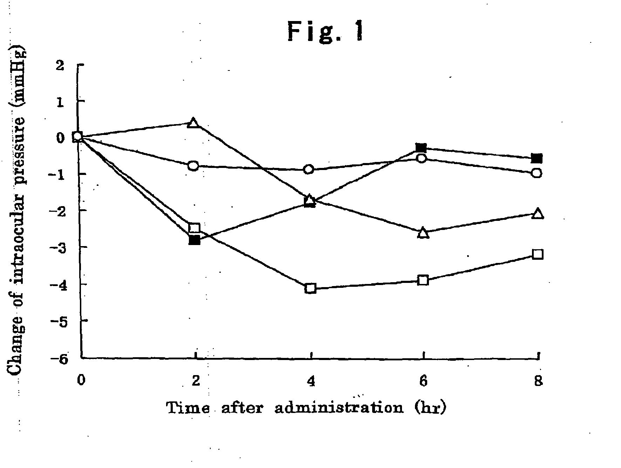

Fig. 1 is a graph showing changes of intraocular pressure with time in respective administration groups. The intraocular pressure is expressed in change from initial intraocular pressure. □ represents a Compound A (said Rho Kinase inhibition) and latanoprost combination administration group, ■ represents a single administration group of Compound A, Δ represents a single administration group of latanoprost, and o represents a control group.

### Best Mode for Carrying out the Invention

A formulation example and pharmacological tests are shown in the following Examples. The Examples are for better understanding of the invention but never limits the scope of the invention.

### Examples

### Formulation Example

This example does not fall into the scope of the claims and does not form part of the invention. It is for purposes of illustration only.

A general formulation example of an ophthalmic solution comprising Rho kinase inhibitor ((R)-(+)-N-(1H-pyrrolo[2,3-b]-pyridin-4-yl)-4-(1-aminoethyl)benzamide dihydrochloride) and prostaglandin in the present invention is shown below.

| | |
|---|---|
| Ophthalmic solution (in 100mL) | |
| (R)=(+)-N-(1H-Pyrrolo[2,3-b]pyridin-4-yl)-4-(1-aminoethyl)benzamide dihydrochloride | 0.3 g |
| Isopropyl unoprostone | 0.06g |
| Boric acid | 0.2 g |
| Concentrated glycerin | 0.25 g |
| Benzalkonium chloride | 0.005 g |
| Diluted hydrochloric acid | quantum sufficient |
| Sodium hydroxide | quantum sufficient |
| Purified water | quantum sufficient |

Ophthalmic solutions having desired combinations and desired concentrations can be prepared by changing the kinds and amounts of Rho kinase inhibitor and prostaglandin and by appropriately changing the amounts of the additives.

### Pharmacological tests

So as to study the utility of the combination of a Rho kinase inhibitor and a prostaglandin, they were administered to Japanese white rabbits (strain: JW, sex: male) or cynomolgus monkeys (Macaca fascicularis, sex: male), examining the effect on reducing intraocular pressure. (R)-(+)-N-(1H-Pyrrolo[2,3-b]pyridin-4-yl)-4-(1-aminoethyl)-benzamide dihydrochloride [Compound A] was used as the Rho kinase inhibitor, latanoprost was used as prostaglandin.

### Preparation of test compound solutions

### 1. Preparation of Rho kinase inhibitor solutions

The Rho kinase inhibitor was dissolved in physiological saline, and then sodium hydroxide was added to the solution to neutralize it (pH 6.0 to 7.0) to thereby prepare Rho kinase inhibitor solutions having desired concentrations.

### 2. Preparation of prostaglandin solutions

A commercially available latanoprost ophthalmic solution (trade name: Xalatan ophthalmic solution) was used as it was, or was diluted with physiological saline to prepare prostaglandin solutions having desired concentrations.

### Method of test

Administering the combination of the Rho kinase inhibitor and prostaglandin, the effect on reducing intraocular pressure was studied. As a reference, administering the Rho kinase inhibitor singly or prostaglandin singly, the effect on reducing intraocular pressure was also studied. As a control, only a vehicle (physiological saline) was administered. As experimental animals, Japanese white rabbits (strain: JW, sex: male) or cynomolgus monkeys (sex: male) were used.

### Method of administration and method of measurement

1. Administration of the combination of a Rho kinase inhibitor and prostaglandin
   1) One drop of a 0.4% oxybuprocaine hydrochloride ophthalmic solution was instilled into both eyes of each experimental animal to anesthetize it topically.
   2) Intraocular pressure was measured immediately before administering the test compound solution, and the intraocular pressure was referred to as initial intraocular pressure.
   3) The Rho kinase inhibitor solution was instilled into one eye of each experimental animal (the other eye was not treated). Since it is impossible to instill the prostaglandin solution at the same time, after a short period (about five minutes), the prostaglandin solution was instilled into the same eye.
   4) Two, four, six and eight hours after instilling the Rho kinase inhibitor solution, one drop of the 0.4% oxybuprocaine hydrochloride ophthalmic solution was instilled into both eyes to anesthetize it topically. Then intraocular pressure was measured three times to obtain the average of three measurements.
2. Administration of a Rho kinase inhibitor alone

Each test was carried out in the same manner as in the above-mentioned combination administration test except that the prostaglandin solution was replaced with physiological saline.

### 3. Administration of a prostaglandin alone

Each test was carried out in the same manner as in the above-mentioned combination administration test except that the Rho kinase inhibitor solution was replaced with physiological saline.

### 4. Control

Each test was carried out in the same manner as in the above-mentioned combination administration test except that the Rho kinase inhibitor solution and the prostaglandin solution were replaced with physiological saline.

### Test 1 The Rho kinase inhibitor solution, the prostaglandin solutions and the experimental animals to be used in the test is shown in Table 1.

Test 1 was carried out according to the above-mentioned method of test, and method of administration and method of measurement.

**Table 1**

| | Rho kinase inhibitor solutions | Prostaglandin solutions | Experimental animals |
|---|---|---|---|
| | | | |
| | | | |
| Test 1 | 0.1% Compound A solution (20 µl) | 0.006% Latanoprost solution (20 µl) | Cynomolgus monkey (three monkeys per group) |
| | | | |

### Results and consideration

Results of Test 1 is shown in Fig. 1 Intraocular pressure is expressed in each change from initial intraocular pressure.

As apparent from Fig. 1 the Rho kinase inhibitor and latanoprost exhibited excellent actions of reducing intraocular pressure compared with administration groups of each drug alone, namely the Rho kinase inhibitor administration or the prostaglandin administration, and exhibited improvement of persistence of the actions. The above-mentioned results show that a stronger reducing effect on intraocular pressure and/or improvement of persistence is obtained by combining the Rho kinase inhibitor with prostaglandin.

### Industrial Applicability

An action on reducing intraocular pressure is increased and/or persistence of the action is improved by administering the Rho kinase inhibitor in combination with the prostaglandin to eyes. Accordingly, the combination is useful as a therapeutic agent for glaucoma.

## Claims

1. A therapeutic agent for the use in the treatment of glaucoma comprising a combination of (R)-(+)-N-(1H-pyrrolo[2,3-b]pyridin-4-yl)-4-(1-aminoethyl)benzamide, and latanoprost.

2. A therapeutic agent for the use in the treatment of glaucoma comprises a combination of (R)-(+)-N-(1H-pyrrolo[2,3-b]pyridin-4-yl)-4-(1-aminoethyl)benzamide, and latanoprost and they complement and/or enhance their actions each other.

3. Use of a combination of, (R)-(+)-N-(1H-pyrrolo[2,3-b]pyridin-4-yl)-4-(1-aminoethyl)benzamide, and latanoprost in the manufacture of a therapeutic agent for glaucoma.

4. Use of a combination of, (R)-(+)-N-(1H-pyrrolo[2,3-b]pyridin-4-yl)-4-(1-aminoethyl)benzamide, and latanoprost in the nanufacture of a therapeutic agent for glaucoma, **characterized in that** their actions are complemented and/or enhance each other.

## Patentansprüche

1. Therapeutisches Mittel zur Verwendung bei der Behandlung eines Glaukoms, umfassend eine Kombination aus (R)-(+)-N-(1H-Pyrrolo[2,3-b]-pyridin-4-yl)-4-(1-aminoethyl)benzamid und Latanoprost.

2. Therapeutisches Mittel zur Verwendung bei der Behandlung eines Glaukoms, umfassend eine Kombination aus (R)-(+)-N-(1H-Pyrrolo[2,3-b]-pyridin-4-yl)-4-(1-aminoethyl)benzamid und Latanoprost, die ihre Wirkungen gegenseitig ergänzen und/oder verstärken.

3. Verwendung einer Kombination aus (R)-(+)-N-(1H-Pyrrolo[2,3-b]pyridin-4-yl)-4-(1-aminoethyl)benzamid und Latanoprost bei der Herstellung eines therapeutischen Mittels gegen Glaukom.

4. Verwendung einer Kombination aus (R)-(+)-N-(1H-Pyrrolo[2,3-b]pyridin-4-yl)-4-(1-aminoethyl)benzamid und Latanoprost bei der Herstellung eines therapeutischen Mittels gegen Glaukom, **dadurch gekennzeichnet, dass** sich deren Wirkungen gegenseitig ergänzen und/oder verstärken.

## Revendications

1. Agent thérapeutique à utiliser dans le traitement du glaucome comprenant une combinaison de (R)-(+)-N-(1H-pyrrolo[2,3-b]pyridin-4-yl)-4-(1-aminoéthyl)benzamide, et de latanoprost.

2. Agent thérapeutique à utiliser dans le traitement du glaucome comprenant une combinaison de (R)-(+)-(N)-(1H-pyrrolo[2,3-b]pyridin-4-yl)-4-(1-aminoéthyl)benzamide, et de latanoprost, dans lequel ces derniers complètent et/ou renforcent leurs actions l'un l'autre.

3. Utilisation d'une combinaison de (R)-(+)-N-(1H-pyrrolo[2,3-b]pyridin-4-yl)-4-(1-aminoéthyl)benzamide, et de latanoprost dans la fabrication d'un agent thérapeutique pour le glaucome.

4. Utilisation d'une combinaison de (R)-(+)-N-(1H-pyrrolo[2,3-b]pyridin-4-yl)-4-(1-aminoéthyl)benzamide, et de latanoprost dans la fabrication d'un agent thérapeutique pour le glaucome, **caractérisé en ce que** leurs actions se complètent et/ou se renforcent l'une l'autre.
